# EUROPEAN PATENT APPLICATION

(11) **EP 3 168 222 A1**
(43) Date of publication of application: **17.05.2017**
(21) Application number: 15819251.8
(22) Date of filing: 08.06.2015
(51) Int. Cl.: C07F 7/18, C07D 493/22

(54) **METHOD FOR PREPARING ERIBULIN INTERMEDIATE**

(30) Priority: 10.07.2014 CN 201410327592
(71) Applicant: Unitris Biopharma Co., Ltd, Pudong District, Shanghai 201203 (CN)
(72) Inventor: ZHANG, Fuyao, Shanghai (CN); LEI, Shenghui, Shanghai (CN); ZHANG, Xinning, Shanghai (CN); GUAN, Zhongjun, Shanghai (CN)
(74) Representative: Gevers & Orès
(86) International application number: PCT/CN2015/080974
(87) International publication number: WO 2016/004805

(57) **Abstract**

The present invention relates to a method for preparing an Eribulin intermediate, and in particular, the invention relates to a compound represented by formula IV, wherein R¹ is a hydroxyl protecting group, preferably a (C₁₋₁₀ alkyl group or aryl group)₃silyl group, and more preferably TBDPS; and R² is a hydroxyl protecting group, preferably a benzyl group or (C₁₋₁₀ alkyl group or aryl group)₃silyl group, and more preferably benzyl group or TBS. The present invention also particularly relates to a method for preparing the compound represented by formula IV. The method has the advantages of a moderate reaction condition, simple and convenient operation and low synthesis cost, and is suitable for mass production.

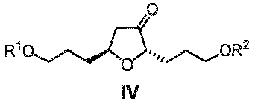

## Description

### FIELD OF THE INVENTION

The present invention relates to a method for preparing Eribulin intermediate

### BACKGROUND OF THE INVENTION

Eribulin (Compound of formula I) is a tubulin inhibitor. It's a derivative of Halichondria B, which is a macrolide extracted from the marine sponge *Halichondria okadai.* On November 15, 2010, the U.S. Food and Drug Administration (FDA) approved eribulin mesylate (Halaven) injection for the treatment of patients with metastatic breast cancer who have received at least two chemotherapy regimens. The action mechanism of eribulin mesylate may be mediated through inhibiting mitosis by directly binding to tubulin, then blocking the growth of microtubules, thereby preventing the growth of cancer cell. As an inhibitor of tubulin polymerization with new action mechanism, eribulin mesylate provides a new therapeutic option for patients with locally advanced breast cancer or metastasis breast cancer to improve their survival rate and quality of life.

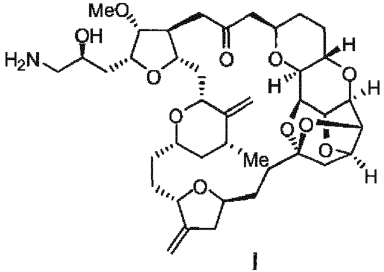

Due to 19 chiral carbon atoms, the total synthesis route and preparation process of eribulin are very complicated, especially the stereoselective control of each chiral center during the process of total synthesis is very challenging.

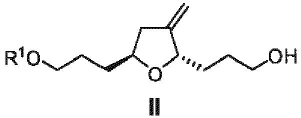

A chiral compound of formula II is a key intermediate to synthesize eribulin. Its synthesis has attracted great interest and attention from chemists, for example, the research group of Professor Kishi at Harvard University in the US reported a synthetic method using a NHK reaction as a key strategy (Org. Lett. 2002, 4, 4435; Org. Lett. 2009, 11, 4520; J. Am. Chem. Soc. 2009, 131, 15636). However, this method is difficult to scale up for industrial production, because the NHK reaction needs to use highly toxic chromium(II) chloride, requires extremely strict anhydrous and oxygen-free reaction condition due to its highly sensitivity to water and oxygen, and has poor repeatability. The research group of Professor Philips at University of Colorado in the US reported another synthetic method adopting Noyori asymmetric hydrogenation and rearrangement of diazoketone as key steps (Angew. Chem. Intl. Ed. 2009, 48, 2346). Although this method is relatively simple, it's difficult to amplify production due to the use of explosive diazomethane and expensive chiral noble metal catalyst. Recently, Alphora Research Inc. in Canada disclosed a method for preparing the compound of formula II using natural sugar as chiral source. However, this synthetic route is tediously long and complicated, and difficult to be applied in industrial production. To sum up, the above reported synthetic methods for the compound of formula II are with harsh reaction conditions, costly, complicated, risky, and therefore not suitable for industrial production.

### DESCRIPTION OF THE INVENTION

To solve the defects of current synthetic methods of the eribulin intermediate of formula **II,** the present invention starts from a readily available chiral chlorinated aldehyde, and applies a mild Aldol reaction and an intramolecular cyclization instead of current NHK reaction with harsh reaction conditions and risky rearrangement of diazoketone. The present invention thus provides a method for preparing eribulin intermediate of formula II, which is with mild reaction conditions and simple procedures, low-cost, and suitable for industrial production.

The present invention provides a novel method for preparing eribulin intermediate of formula **II,** 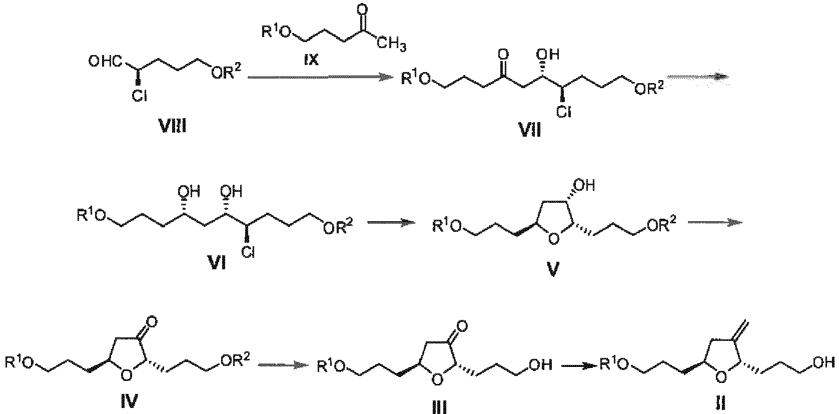 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

Specifically, the preparation method comprises the following steps of:
1) reacting a compound of formula **VIII** with a compound of formula **IX** via an Aldol reaction to obtain a compound of formula **VII;** wherein the reaction is preferably conducted in the presence of a base (e.g., lithium diisopropylamide);
2) obtaining a compound of formula **VI** from the compound of formula **VII** via a chirality-induced reduction reaction; wherein the reductant is preferably alkylaluminum hydride;
3) obtaining a compound of formula **V** from the compound of formula **VI** via an intramolecular cyclization reaction; wherein the intramolecular cyclization is preferably conducted in the presence of silver(II) oxide and silver trifluoromethanesulfonate;
4) oxidizing a hydroxyl group of the compound of formula **V** to obtain a compound of formula **IV;** wherein for example the oxidant is preferably selected from Dess-Martin reagent and Swern reagent;
5) selectively removing one of the hydroxyl protecting groups of the compound of formula **IV** to obtain a compound of formula **III**;
6) obtaining the compound of formula **II** from the compound of formula **III** via a Wittig reaction; wherin the reaction is preferably conducted under a basic condition, and the reaction reagent is preferably methyltriphenylphosphonium halide.

Wherein the compound of formula **VIII** can be prepared according to a reference (Angew. Chem. Intl. Ed. 2009, 48, 5121).

In a preferred embodiment of the present invention, the hydroxyl protecting group R¹ is TBDPS, and the hydroxyl protecting group R² is benzyl.

In another preferred embodiment of the present invention, the hydroxyl protecting group R¹ is TBDPS, and the hydroxyl protecting group R² is TBS.

In a particularly preferred embodiment, the present invention provides a synthetic route for a compound of formula **IIa** as below:

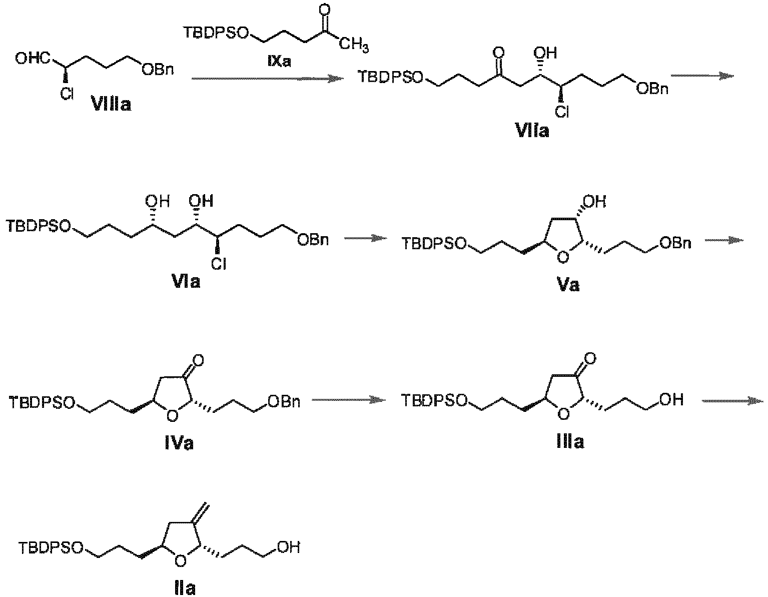

Specifically, the method comprises the following steps of:
1) reacting a chiral chlorinated aldehyde of formula **VIIIa** with a compound of formula **IXa** via an Aldol reaction in the presence of lithium diisopropylamide to obtain a compound of formula **VIIa;**
2) obtaining a compound of formula **VIa** from the compound of formula **VIIa** via a chirality-induced reduction reaction with diisobutyl aluminium hydride;
3) obtaining a compound of formula **Va** from the compound of formula **VIa** via an intramolecular cyclization reaction in the presence of silver(II) oxide and silver trifluoromethanesulfonate;
4) oxidizing an unprotected hydroxyl group of the compound of formula **Va** to a keto group by Dess-Martin reagent, and obtaining a compound of formula **IVa;**
5) selectively removing one of the hydroxyl protecting groups of the compound of formula **IVa** to obtain a compound of formula **IIIa;**
6) obtaining the compound of formula **IIa** from the compound of formula **IIIa** via a Wittig reaction with methyltriphenylphosphonium bromide under a basic condition.

Wherein the chiral chlorinated aldehyde of formula **VIIIa** can be prepared according to a reference (Angew. Chem. Intl. Ed. 2009, 48, 5121).

The present invention also provides the compound of formula **VII,** 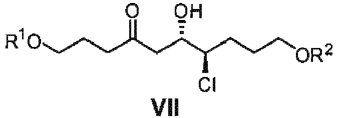 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

In a preferred embodiment of the present invention, the hydroxyl protecting group R¹ is TBDPS, and the hydroxyl protecting group R² is benzyl.

In another preferred embodiment of the present invention, the hydroxyl protecting group R¹ is TBDPS, and the hydroxyl protecting group R² is TBS.

The present invention further provides a preparation method of the compound of formula **VII,** wherein the compound of formula **VIII** is reacted with the compound of formula **IX** via an Aldol reaction to obtain the compound of formula **VII,** and the reaction is preferably conducted in the presence of a base (e.g., lithium diisopropylamide), 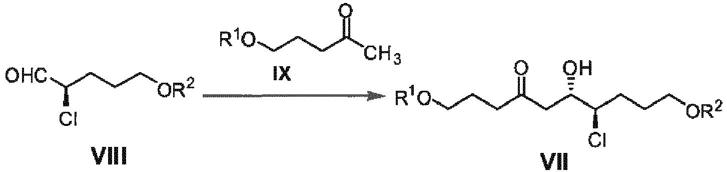 wherein R¹ and R² are as defined in the compound of formula **VII.**

The present invention also provides the compound of formula **VI,** 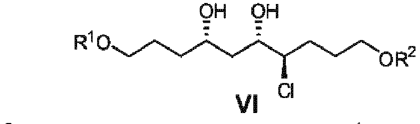 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

In a preferred embodiment of the present invention, the hydroxyl protecting group R¹ is TBDPS, and the hydroxyl protecting group R² is benzyl.

In another preferred embodiment of the present invention, the hydroxyl protecting group R¹ is TBDPS, and the hydroxyl protecting group R² is TBS.

The present invention further provides a method for preparing the compound of formula **VI,** wherein the compound of formula **VI** is prepared from the compound of formula **VII** via a chirality-induced reduction reaction, and the reductant is preferably alkylaluminum hydride, 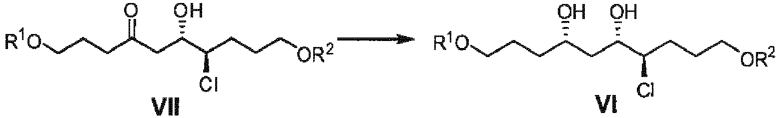 wherein R¹ and R² are as defined in the compound of formula **VI.**

The present invention also provides the compound of formula **V,** 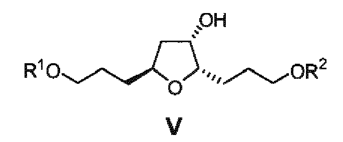 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

In a preferred embodiment of the present invention, the hydroxyl protecting group R¹ is TBDPS, and the hydroxyl protecting goup R² is benzyl.

In another preferred embodiment of the present invention, the hydroxyl protecting group R¹ is TBDPS, and the hydroxyl protecting group R² is TBS.

The present invention further provides a method for preparing the compound of formula **V**, wherein the compound of formula **V** is prepared from the compound of formula **VI** via an intramolecular cyclization reaction, and the intramolecular cyclization is preferably conducted in the presence of silver(II) oxide and silver trifluoromethanesulfonate, 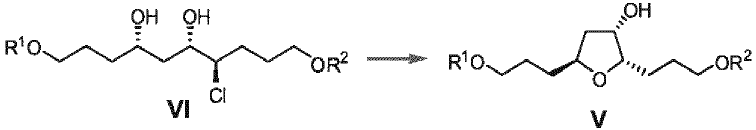 wherein R¹ and R² are as defined in the compound of formula V.

The present invention also provides the compound of formula IV, 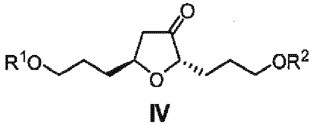 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

In a preferred embodiment of the present invention, the hydroxyl protecting group R¹ is TBDPS, and the hydroxyl protecting group R² is benzyl.

In another preferred embodiment of the present invention, the hydroxyl protecting group R¹ is TBDPS, and the hydroxyl protecting group R² is TBS.

The present invention further provides a method for preparing a compound of formula **IV,** wherein a hydroxyl group of the compound of formula **V** is oxidized to prepare the compound of formula **IV,** and for example, the oxidant is preferably selected from Dess-Martin reagent and Swern reagent, 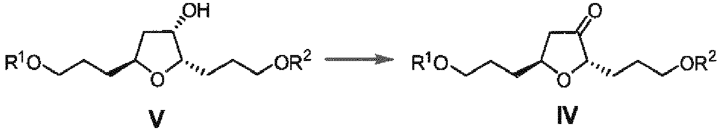 wherein R¹ and R² are as defined in the compound of formula **IV.**

The compound of formula **IV** can be prepared from the compound of formula **II** via a relatively shorter route, which comprises the following specific steps of:
1) obtaining the compound of formula **V** from the compound of formula **VI** via an intramolecular cyclization reaction; wherein the intramolecular cyclization is preferably conducted in the presence of silver(**II**) oxide and silver trifluoromethanesulfonate;
2) oxidizing a hydroxyl group of the compound of formula **V** to obtain the compound of formula **IV;** wherein for example the oxidant is preferably selected from Dess-Martin reagent and Swern reagent;
3) removing a hydroxyl protecting group of the compound of formula **IV,** and then obtaining the compound of formula **II** via a Wittig reaction, wherein the reaction is preferably under a basic condition, and the reaction reagent is preferably methyltriphenylphosphonium halide, 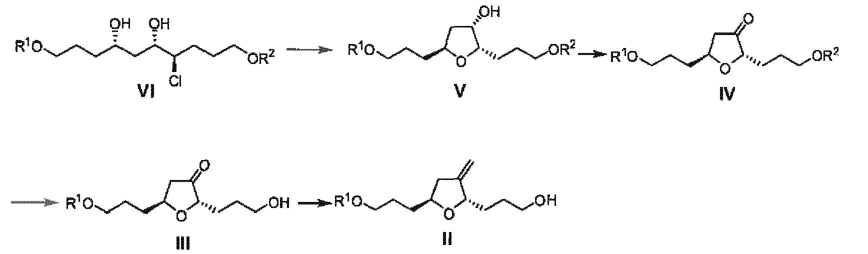 wherein R¹ and R² are hydroxyl protecting groups, R¹ is preferably a (C₁₋₁₀ alkyl or aryl)₃silyl group, more preferably TBDPS; and R² is preferably benzyl or a (C₁₋₁₀ alkyl or aryl)₃silyl group, more preferably benzyl.

In a preferred embodiment of the present invention, the hydroxyl protecting group R¹ is TBDPS, and the hydroxyl protecting group R² is benzyl.

In another preferred embodiment of the present invention, the hydroxyl protecting group R¹ is TBDPS, and the hydroxyl protecting group R² is TBS.

If some intermediates of the above steps are commercial available, the compound of formula **II** can be prepared via shorter routes. For example, the intermediate of formula **V** or **IV** can be purchased, and then the compound of formula **II** can be prepared from this intermediate according to the above methods.

Furthermore, the present invention also provides a preparation method of eribulin, comprising preparing the compound of formula **II** or **IIa** according to the previously mentioned methods of the present invention, and then preparing eribulin from the compound of formula **II** or **IIa** according to known methods. The known methods can refer to the following literatures: Org. Lett. 2002, 4, 4435; Org. Lett. 2009, 11, 4520; J. Am. Chem. Soc. 2009, 131, 15636; Angew. Chem. Intl. Ed. 2009, 48, 2346; Synlett. 2013, 24, 323; Synlett. 2013, 24, 327; Synlett. 2013, 24, 333.

Unless otherwise defined, the terms used herein have the following meanings:

"Alkyl" refers to saturated aliphatic hydrocarbon groups, including linear and branched chains containing from 1 to 10 carbon atoms, preferably from 1 to 6 carbon atoms. Representative examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group may be substituted or unsubstituted. When substituted, the substituent group(s) can be substituted at any available connection point, and preferably the substituent group(s) is one or more groups independently selected from the group consisting of alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkoxy, heterocycloalkoxy, cycloalkylthio, heterocycloalkylthio, and oxo group.

The hydroxyl protecting groups used in the present invention are appropriate hydroxyl protecting groups known in the field of the invention. See the hydroxyl protecting groups described in "Protective Groups in Organic Synthesis", 5Th Ed. T. W. Greene & P. G. M. Wuts. For example, the hydroxyl protecting groups may be preferably (C₁₋₁₀ alkyl or aryl)₃silyl groups, e.g. triethylsilyl, triisopropylsilyl, tert-butyldimethylsilyl, tert-butyldiphenylsilyl, etc. The hydroxyl protecting groups also may be C₁₋₁₀ alkyl or substituted alkyl groups, e.g. methyl, tert-butyl, allyl, benzyl, methoxymethyl, ethoxyethyl, 2-tetrahydropyranyl (THP), etc. The hydroxyl protecting groups also may be (C₁₋₁₀ alkyl or aryl)acyl groups, e.g. formyl, acetyl, benzoyl, etc. The hydroxyl protecting groups also may be (C₁₋₆ alkyl or C₆₋₁₀ aryl)sulfonyl groups or (C₁₋₆ alkoxyl or C₆₋₁₀ aryloxy) carbonyl groups.

"Aryl" refers to a 6- to 14-membered all-carbon monocyclic ring or polycyclic fused ring (a fused ring system means that each ring in the system shares an adjacent pair of carbon atoms with another ring in the system) with conjugated π-electron system, preferably 6- to 10-membered, more preferably phenyl group and naphthyl group, most preferably phenyl group. The aryl group may be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkoxy, heterocycloalkoxy, cycloalkylthio, and heterocycloalkylthio.

**List of Abbreviations:**

| Abbreviations | Full names |
|---|---|
| Bn | benzyl |
| TBDPS | tert-butyldiphenylsilyl |
| TBS | tert-butyldimethylsilyl |
| LDA | lithium diisopropylamide |
| DIBAL-H | diisobutyl aluminium hydride |

Structural Formulas of Compounds Disclosed in the Examples in the Table Below

| Compounds | Structural Formulas | Compounds | Structural Formulas |
|---|---|---|---|
| IIa | 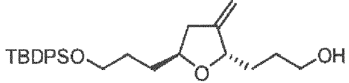 | | |
| IIIa | 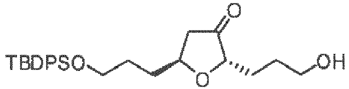 | | |
| IVa | 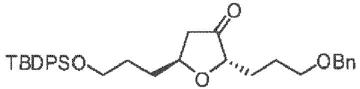 | IVb | 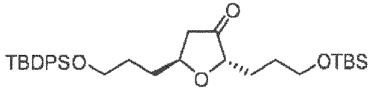 |
| Va | 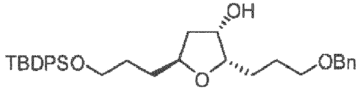 | Vb | 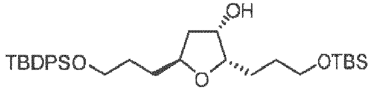 |
| VIa | 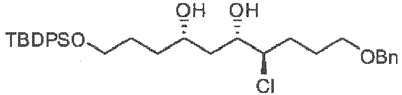 | VIb | 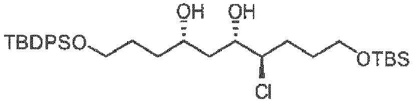 |
| VIIa | 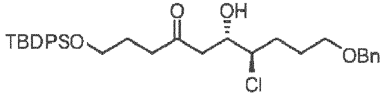 | VIIb | 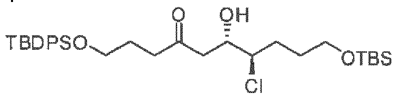 |
| VIIIa | 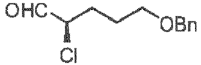 | VIIIb | 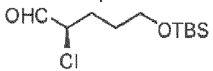 |
| IXa | 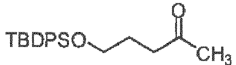 | | |

### PREFERRED EMBODIMENTS

The present invention is described in detail with reference to the following specific embodiments so that the technicians in the art will understand the present invention in a more comprehensive manner. The specific embodiments are used only to illustrate the technical solution of the present invention, but are not used to limit the scope of the present invention in any way.

### Example 1

### Preparation of the compound of formula VIIa

The compound of formula **IXa** (13.1 g, prepared according to Tetrahedron Lett. 2001, 42, 9233) was dissolved in tetrahydrofuran (240 mL). The reaction mixture was cooled to -10 °C, and then a solution of LDA in tetrahydrofuran (46 mL, 1.0 M) was added dropwise. The reaction mixture was stirred at -10 °C for 2 hrs, and then the compound of formula **VIIIa** (6.5 g, prepared according to Angew. Chem. Intl. Ed. 2009, 48, 5121) was added. After the reaction mixture was stirred at -10 °C for another 2 hrs, it was quenched with a saturated aqueous solution of ammonium chloride (56 mL) and then extracted with 200 mL ethyl acetate. The organic layer was separated, dried over anhydrous sodium sulfate, concentrated, and then purified by column chromatography to obtain the compound of formula **VIIa** (14.2 g).
¹H NMR (CDCl₃, 400 MHz) *δ* = 7.68-7.64 (m, 4 H), 7.46-7.32 (m, 11 H), 4.51 (s, 2H), 4.10-4.07 (m, 1 H), 3.97-3.86 (m, 3 H), 3.73-3.67 (m, 2 H), 3.55-3.49 (m, 2 H), 1.05 (s, 9 H).

### Example 2

### Preparation of the compound of formula VIa

The compound of formula **VIIa** (12.2 g) was dissolved in tetrahydrofuran (330 mL). The reaction mixture was cooled to -30 °C, and then a solution of DIBAL-H in hexane (66 mL, 1.0 M solution) was added dropwise. After the reaction mixture was stirred at -30 °C for 5 hrs, it was quenched with 8 mL methanol and 160 mL a saturated aqueous solution of potassium sodium tartrate, and then extracted with 300 mL ethyl acetate. The organic layer was separated, dried over anhydrous sodium sulfate, concentrated, and then purified by column chromatography to obtain the compound of formula **VIa** (9.6 g).
¹H NMR (CDCl₃, 400 MHz) *δ* = 7.68-7.64 (m, 4 H), 7.46-7.32 (m, 11 H), 4.51 (s, 2H), 4.10-4.07 (m, 1 H), 3.97-3.86 (m, 3 H), 3.73-3.67 (m, 2 H), 3.55-3.49 (m, 2 H), 1.05 (s, 9 H).

### Example 3

### Preparation of the compound of formula Va

The compound of formula **VIa** (8.0 g) was dissolved in tetrahydrofuran (300 mL). The reaction mixture was cooled to 0 °C, and then silver(II) oxide (3.2 g) and silver trifluoromethanesulfonate (3.5 g) were added. After the reaction mixture was stirred at 20 °C for 18 hrs, it was quenched with a saturated aqueous solution of sodium bicarbonate, and then extracted with 200 mL ethyl acetate. The organic layer was separated, dried over anhydrous sodium sulfate, concentrated, and then purified by column chromatography to obtain the compound of formula **Va** (6.9 g).
¹H NMR (CDCl₃, 400 MHz) *δ* = 7.68-7.64 (m, 4 H), 7.42-7.32 (m, 11 H), 4.51 (s, 1.6 H), 4.50 (s, 0.4 H), 4.25-4.15 (m, 1.6 H), 4.05-3.92 (m, 0.8 H), 3.79-3.74 (td, *J* = 2.9, 6.9 Hz, 1 H), 3.70-3.65(m, 2 H), 3.56-3.47 (m, 2 H), 2.09-1.99 (m, 2H), 1.04 (s, 9H).

### Example 4

### Preparation of the compound of formula IVa

The compound of formula **Va** (11.5 g) was dissolved in dichloromethane (100 mL). The reaction mixture was cooled to 0 °C, and then sodium bicarbonate (7.2 g) and Dess-Martin periodinane (10.9 g) were added. After the reaction mixture was stirred at 20 °C for 1 hrs, it was quenched with a saturated brine, and then extracted with 500 mL methyl tert-butyl ether. The organic layer was separated, dried over anhydrous sodium sulfate, concentrated, and then purified by column chromatography to obtain the compound of formula **IVa** (11.1 g).
¹H NMR (CDCl₃, 400 MHz) *δ* = 7.68-7.64 (m, 4 H), 7.42-7.32 (m, 11 H), 4.49 (s, 1.6 H), 4.33-4.25 (m, 1 H), 3.69 (t, *J=* 5.7 Hz, 2 H), 3.48 (t, *J*= 6.1 Hz, 2 H), 2.53 (dd, *J=* 6.9, 18.2 Hz, 1 H), 2.22-2.14 (m, 1 H), 1.79-1.57 (m, 9 H), 1.04 (s, 9H).

### Example 5

### Preparation of the compound of formula IIIa

The compound of formula **IVa** (4.0 g) was dissolved in tetrahydrofuran (100 mL). The reaction mixture was added with Pd/C (0.4 g, 10 wt%), and stirred under 1.5 atm hydrogen at 50 °C for 12 hrs. The reaction mixture was filtrated. The filtrate was concentrated, and then purified by column chromatography to obtain the compound of formula **IIIa** (2.8 g).
¹H NMR (400 MHz, CDCl₃): δ = 7.68-7.64 (m, 4 H), 7.45-7.35 (m, 6 H), 4.38-4.30 (m, 0.33 h), 4.19-4.09 (m, 1 H), 3.95-3.91 (dd, *J* = 4.4, 8.9 Hz, 0.33 H), 3.88-3.80 (m, 1.3 H), 3.73-3.62 (m, 4 H).

### Example 6

### Preparation of the compound of formula IIa

Methyltriphenylphosphonium bromide (4.7 g) was dissolved in tetrahydrofuran (100 mL). The reaction mixture was cooled to -50 °C, and then a solution of potassium tert-butoxide in tetrahydrofuran (13 mL, 1.0 M) was added dropwise. The reaction mixture was stirred at -10 °C for 1 hrs, and then a solution of the compound of formula **IIIa** (4.0 g) in tetrahydrofuran (50 mL) was added dropwise. After the reaction mixture was stirred at -10 °C for another 4 hrs, it was quenched with 50 mL a saturated aqueous solution of ammonium chloride and 50 mL a saturated brine, and then extracted with 200 mL ethyl acetate. The organic layer was separated, dried over anhydrous sodium sulfate, concentrated, and then purified by column chromatography to obtain the compound of formula **IIa** (3.7 g).
¹H NMR (CDCl₃, 400 MHz) *δ* = 7.66-7.64 (m 4 H), 7.44-7.35 (m, 6 H), 4.99 (dd, *J* = 2.0, 4.4, 1 H), 4.85 (dd, *J =* 2.4, 4.4 Hz, 1 H), 4.41-4.36 (m, 1 H), 4.07-4.00 (m, 1 H), 3.72-3.60 (m, 4 H), 2.69-2.63 (m, 1 H), 2.56-2.53 (m, 1 H), 2.29-2.23 (m, 1 H), 1.77-1.51 (m, 8 H), 1.04 (s, 9 H).

### Example 7

### Preparation of the compound of formula VIIb

The compound of formula **IXa** (13.1 g, prepared according to Tetrahedron Lett. 2001, 42, 9233) was dissolved in tetrahydrofuran (240 mL). The reaction mixture was cooled to -10 °C, and then a solution of LDA in tetrahydrofuran (46 mL, 1.0 M) was added dropwise. The reaction mixture was stirred at -10 °C for 2 hrs, and then the compound of formula **VIIIb** (7.2 g, prepared according to Angew. Chem. Intl. Ed. 2009, 48, 5121) was added. After the reaction mixture was stirred at -10 °C for another 2 hrs, it was quenched with a saturated solution of ammonium chloride (56 mL) and then extracted with 200 mL ethyl acetate. The organic layer was separated, dried over anhydrous sodium sulfate, concentrated, and then purified by column chromatography to obtain the compound of formula **VIIb** (14.6 g).
¹H NMR (CDCl₃, 400 MHz)) *δ* = 7.67-7.63 (m, 4 H), 7.45-7.36 (m, 6 H), 4.15-4.08 (m, 1 H), 3.95-3.91 (m, 1 H), 3.71-3.63 (m, 4 H), 3.30 (d, *J=* 5.0 Hz, 1 H), 2.85-2.70 (m, 2 H), 2.59 (t, J = 7.3 Hz, 2 H), 2.07-1.97 (m, 1 H), 1.89-1.78 (m, 3 H), 1.74-1.57 (m, 2 H), 1.05 (s, 9 H), 0.90 (s, 9 H), 0.06 (s, 6 H).

### Example 8

### Preparation of the compound of formula VIb

The compound of formula **VIIb** (12.7 g) was dissolved in tetrahydrofuran (330 mL). The reaction mixture was cooled to -30 °C, and then a solution of DIBAL-H in hexane (66 mL, 1.0 M solution) was added dropwise. After the reaction mixture was stirred at -30 °C for 5 hrs, it was quenched with 8 mL methanol and 160 mL a saturated aqueous solution of potassium sodium tartrate, and then extracted with 300 mL ethyl acetate. The organic layer was separated, dried over anhydrous sodium sulfate, concentrated, and then purified by column chromatography to obtain the compound of formula **VIb** (9.9 g).
¹H NMR (CDCl₃, 400 MHz) *δ* = 7.70-7.46 (m, 4 H), 7.44-7.38 (m, 6 H), 3.97-3.90 (m, 3 H), 3.72-3.66 (m, 4 H), 2.08-1.93 (m, 1 H), 1.87-1.50 (m, 9 H), 1.07 (s, 9 H), 0.91 (s, 9 H), 0.08 (s, 6 H).

### Example 9

### Preparation of the compound of formula Vb

The compound of formula **VIb** (8.5 g) was dissolved in tetrahydrofuran (300 mL). The reaction mixture was cooled to 0 °C, and then silver(II) oxide (3.2 g) and silver trifluoromethanesulfonate (3.5 g) were added. After the reaction mixture was stirred at 20 °C for 18 hrs, it was quenched with a saturated aqueous solution of sodium bicarbonate, and then extracted with 200 mL ethyl acetate. The organic layer was separated, dried over anhydrous sodium sulfate, concentrated, and then purified by column chromatography to obtain the compound of formula **Vb** (7.2 g).
¹H NMR (CDCl₃, 400 MHz) *δ* = 7.70-7.44 (m, 4 H), 7.41-7.35 (m, 6 H), 4.26-4.22 (m, 1 H), 4.21-4.18 (m, 1 H), 3.80-3.77 (m, 1 H), 3.71-3.65 (m, 4 H), 2.27 (d, *J=* 4.8 Hz, 1 H), 2.09 (dd, *J* = 6.0, 13.2 Hz, 1H), 1.74-1.50 (m, 10 H), 1.05 (s, 9H), 0.91 (s, 9 H), 0,08 (s, 6 H).

### Example 10

### Preparation of the compound of formula IVb

The compound of formula **Vb** (11.9 g) was dissolved in dichloromethane (100 mL). The reaction mixture was cooled to 0 °C, and then sodium bicarbonate (7.2 g) and Dess-Martin periodinane (10.9 g) were added. After the reaction mixture was stirred at 20 °C for 1 hrs, it was quenched with a saturated brine, and then extracted with 500 mL methyl tert-butyl ether. The organic layer was separated, dried over anhydrous sodium sulfate, concentrated, and then purified by column chromatography to obtain the compound of formula **IVb** (11.3 g).
¹H NMR (CDCl₃, 400 MHz) *δ* = 7.67-7.65 (m, 4 H), 7.45-7.36 (m, 6 H), 4.32-4.29 (m, 1 H), 3.93-3.90 (m, 1 H), 3.71 (t, *J=* 5.6 Hz, 2 H), 3.63 (t, *J=* 6.0 Hz, 2 H), 2.54 (dd, *J=* 6.4, 17.6 Hz, 1H), 2.19 (dd, *J*= 7.2, 18.0 Hz, 1 H), 1.80-1.55 (m, 8 H), 1.05 (s, 9 H), 0.88 (s, 9 H), 0.04 (s, 6 H).

### Example 11

### Preparation of the compound of formula IIIa

The compound of formula **IVb** (4.6 g) was dissolved in methanol (100 mL), and then pyridinium 4-toluenesulfonate (0.6 g) was added. After the reaction mixture was stirred at 20 °C for 22 hrs, it was quenched with a saturated aqueous solution of sodium bicarbonate, and then extracted with 200 mL ethyl acetate. The organic layer was separated, dried over anhydrous sodium sulfate, concentrated, and then purified by column chromatography to obtain the compound of formula **IIIa** (3.1 g).
¹H NMR (400 MHz, CDCl₃): δ = 7.68-7.64 (m, 4 H), 7.45-7.35 (m, 6 H), 4.38-4.30 (m, 0.33 h), 4.19-4.09 (m, 1 H), 3.95-3.91 (dd, J = 4.4, 8.9 Hz, 0.33 H), 3.88-3.80 (m, 1.3 H), 3.73-3.62 (m, 4 H).

Since the present invention has been described based on the specific embodiments thereof, some modifications and equivalent variations that are apparent to those skilled in the art are also within the scope of the present invention.

## Claims

1. A compound of formula **IV:** 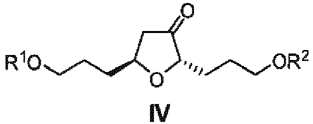 wherein R¹ is a hydroxyl protecting group, preferably a (C₁₋₁₀ alkyl or aryl)₃silyl group, more preferably TBDPS; and R² is a hydroxyl protecting group, preferably benzyl or a (C₁₋₁₀ alkyl or aryl)₃silyl group, more preferably benzyl or TBS.

2. A method for preparing a compound of formula **IV, characterized in that** a hydroxyl group of a compound of formula **V** is oxidized to prepare the compound of formula **IV,** 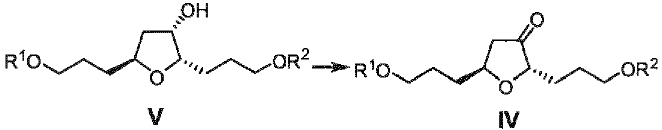 wherein R¹ and R² are as defined in claim 1.

3. A compound of formula **V:** 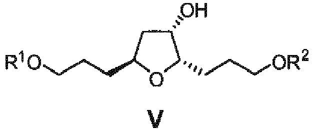 wherein R¹ and R² are as defined in claim 1.

4. A method for preparing a compound of formula **V, characterized in that** the compound of formula **V** is prepared from a compound of formula **VI** via an intramolecular cyclization reaction, 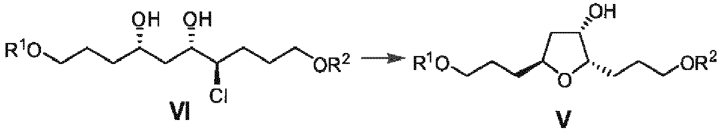 wherein R¹ and R² are as defined in claim 1.

5. A method for preparing a compound of formula **II, characterized in that** it comprises the following steps of:
1) removing a hydroxyl protecting group of a compound of formula **IV** to obtain a compound of formula **III**;
2) obtaining the compound of formula **II** from the compound of formula **III** via a Wittig reaction; 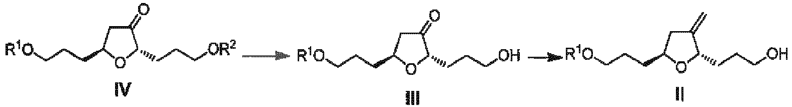 wherein R¹ and R² are as defined in claim 1.

6. The method for preparing a compound of formula **II** according to claim 5, **characterized in that** it further comprises a step of oxidizing a hydroxyl group of a compound of formula V to obtain the compound of formula **IV,** 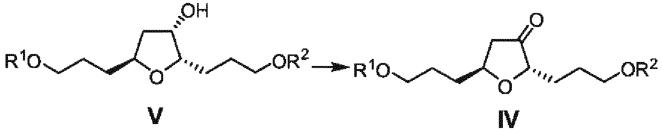 wherein R¹ and R² are as defined in claim 1.

7. The method for preparing a compound of formula **II** according to claim 6, **characterized in that** it further comprises a step of preparing the compound of formula **V** from a compound of formula **VI** via an intramolecular cyclization reaction, 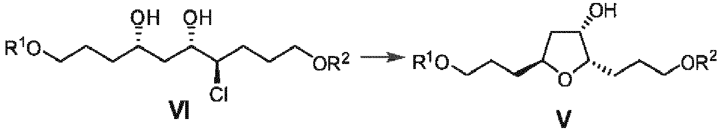 wherein R¹ and R² are as defined in claim 1.

8. A compound of formula **VI:** 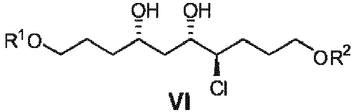 wherein R¹ and R² are as defined in claim 1.

9. A method for preparing a compound of formula **VI, characterized in that** the compound of formula **VI** is prepared from a compound of formula **VII** via a chirality-induced reduction reaction, 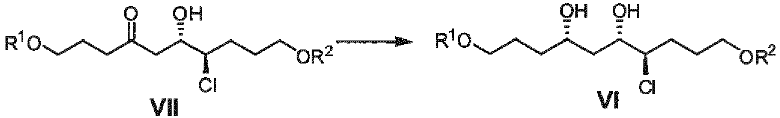 wherein R¹ and R² are as defined in claim 1.

10. A compound of formula **VII:** 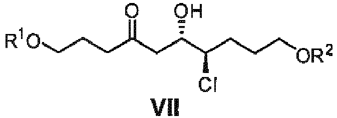 wherein R¹ and R² are as defined in claim 1.

11. A method for preparing a compound of formula **VII, characterized in that** a compound of formula **VIII** is reacted with a compound of formula **IX** via an Aldol reaction to prepare the compound of formula **VII,** 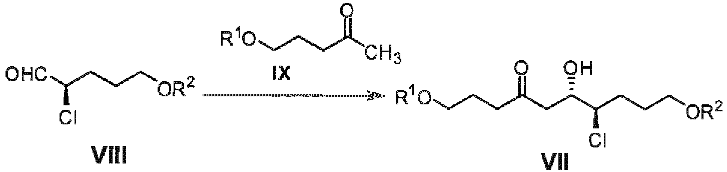 wherein R¹ and R² are as defined in claim 1.

12. A method for preparing a compound of formula **II, characterized in that** it comprises the following steps of: 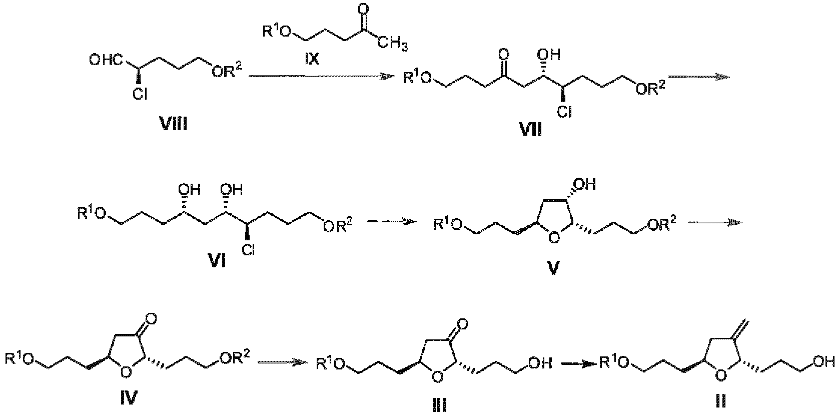
1) reacting a compound of formula **VIII** with a compound of formula **IX** via an Aldol reaction to obtain a compound of formula **VII;**
2) obtaining a compound of formula **VI** from the compound of formula **VII** via a chirality-induced reduction reaction;
3) obtaining a compound of formula **V** from the compound of formula **VI** via an intramolecular cyclization reaction;
4) oxidizing a hydroxyl group of the compound of formula **V** to obtain a compound of formula **IV;**
5) removing a hydroxyl protecting group of the compound of formula **IV** to obtain a compound of formula **III**;
6) obtaining the compound of formula II from the compound of formula **III** via a Wittig reaction;
wherein R¹ and R² are as defined in claim 1.

13. A method for preparing a compound of formula **IIa, characterized in that** it comprises the following steps of: 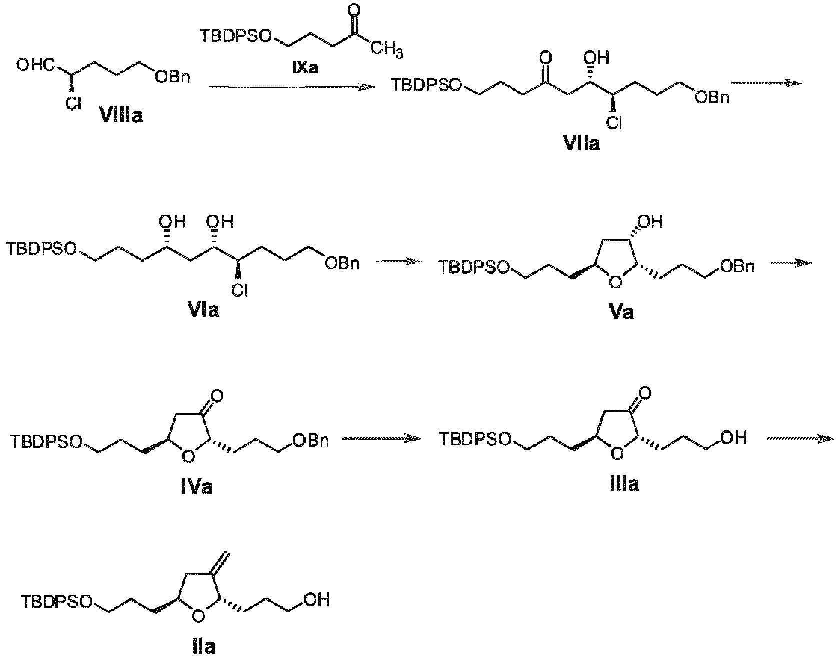
1) reacting a compound of formula **VIIIa** with a compound of formula **IXa** via an Aldol reaction to obtain a compound of formula **VIIa;**
2) obtaining a compound of formula **VIa** from the compound of formula **VIIa** via a chirality-induced reduction reaction;
3) obtaining a compound of formula **Va** from the compound of formula **VIa** via an intramolecular cyclization reaction;
4) oxidizing a hydroxyl group of the compound of formula **Va** to obtain a compound of formula **IVa;**
5) removing a hydroxyl protecting group of the compound of formula **IVa** to obtain a compound of formula **IIIa;**
6) obtaining the compound of formula **IIa** from the compound of formula **IIIa** via a Wittig reaction.

14. A method for preparing eribulin, **characterized in that** it comprises the steps of preparing a compound of formula **II** according to the method of any one of claims 5-7 and 12, or preparing a compound of formula **IIa** according to the method of claim 13, and then preparing eribulin from the compound of formula **II** or **IIa.**
